# FASCICULE DE BREVET EUROPEEN

(11) **EP 0 983 055 B1**
(45) Date de publication et mention de la délivrance du brevet: **14.04.2004**
(21) Numéro de dépôt: 98958280.4
(22) Date de dépôt: 30.11.1998
(51) Int. Cl.: A61K 7/48, A61K 7/06, A61K 31/00

(54) **COMPOSITION COMPORTANT UN N-UNDECYLENOYL AMINOACIDE; APPLICATION EN COSMETIQUE**
EINE N-UNDECYLENOYL-AMINOSAURE ENTHALTENDE ZUSAMMENSETZUNG; ANWENDUNG IN DER KOSMETIK
COMPOSITION COMPRISING AN AMINO ACID N-UNDECYLENOYL; APPLICATION IN COSMETICS

(30) Priorité: 01.12.1997 FR 9715087
(43) Date de publication de la demande: 08.03.2000
(73) Titulaire: SOCIETE D'EXPLOITATION DE PRODUITS POUR LES INDUSTRIES CHIMIQUES, S.E.P.P.I.C., 75321 Paris Cédex 07 (FR)
(72) Inventeur: STOLTZ, Corinne, F-75012 Paris (FR)
(74) Mandataire: Conan, Philippe Claude
(86) Numéro de dépôt international: PCT/FR1998/002571
(87) Numéro de publication internationale: WO 1999/027902

(56) Documents cités:
- DE-A- 2 203 228
- FR-A- 2 747 309
- BASE DE DONN ES "CHEMICAL ABSTRACTS" (SERVEUR: STN); Abrégé 82: 103 149, Colombus, OH, USA; & JP 49 093 521 (MORINAGA MILK IND. CO., Ltd) 5 septembre 1974 XP002091434

## Description

L'invention a pour objet une composition anti-microbienne, son utilisation dans la préparation de compositions cosmétiques et les compositions ainsi obtenues.

La plupart des problèmes liés à la peau tels que les pellicules, les comédons, les kystes, les « points noirs », ou toutes autres manifestations affectant l'esthétique du corps humain, sont liés à la présence de germes bactériens qui, notamment à cause de leurs enzymes, induisent une réaction de celle-ci comme par exemple, une inflammation.

Parmi ces problèmes de peau, on peut citer l'acné, qui est une affection cutanée fréquente touchant, à la date de dépôt de la présente demande de brevet, environ cinq millions de personnes dans ce pays; la forme la plus fréquente est l'acné polymorphe juvénile qui apparaît à la puberté.

L'acné se déclenche au niveau du follicule pilo-sébacé en provoquant simultanément l'hyperkératinisation du canal pilo-sébacé, l'hypersécrétion sébacée ainsi que la prolifération bactérienne au niveau du follicule pilo-sébacé

L'hyperkératinisation du canal pilo-sébacé conduit à son obstruction, qui à son tour favorise l'implantation d'un germe retrouvé fréquemment dans cette pathologie: le Propionibacterium acnes.

L'hypersécrétion sébacée est un facteur constant au cours de l'acné; elle provient d'une sensibilité accrue de la glande sébacée aux androgènes, ce qui conduit à la sécrétion plus importante de sébum. En raison de l'hyperkératinisation du canal pilo-sébacé, l'élimination du sébum est gênée, voire empêchée, et cela induit la formation de comédons et de microkystes fermés. Le sébum ainsi accumulé dans un follicule devient alors un lieu de prolifération bactérienne. Le sébum, riche en triglycérides, est alors très rapidement dégradé en acides gras libres (AGL) par les lipases provenant des germes bactériens. Les acides gras libres ainsi formés, s'oxydent au contact de l'oxygène de l'air, notamment en peroxydes, qui entretiennent, voir aggravent, l'inflammation locale.

Que ce soit sur une peau saine ou acnéique, la flore bactérienne que l'on rencontre à la surface de celle-ci ou à l'intérieur des comédons est qualitativement la même: il s'agit de levures telles que Pityrosporum ovale et Pityrosporum orbiculare, de staphylocoques tels que Staphylococus epidermis, Staphylococus capitis ou Staphylococus hominis ou encore de propionibactéries telle que Propionibacterium acnes.

Propionibacterium acnes produit des lipases qui sont capables d'hydrolyser les triglycérides du sébum en acides gras libres. Les acides gras libres sont connus pour être comédogènes, c'est à dire qu'il peuvent provoquer une hyperkératose folliculaire. Ainsi, la colonisation du follicule par ces bactéries détermine une autre source de matière comédogènique qui, par la suite, provoquera le développement de micro-comédons. Propionibacterium acnes est aussi responsable de l'accumulation de leucocytes neutrophiles et indirectement de lymphocytes mononucléaires, entraînant ainsi le développement d'une inflammation et la mise en jeu d'une réponse immunitaire.

Les staphylocoques précédemment nommés colonisent aussi bien les peaux saines que les lésions acnéiques, dans lesquelles on les rencontre avec une fréquence comparable à celle de Propionibacterium acnes ; 70 à 75% des comédons sont colonisés, soit par des staphyloccoques, soit par des propionibactéries avec en moyenne 10⁴ à 10⁵ bactéries par comédons. Le fait que les comédons soient ouverts (points noirs) ou fermés (microkystes) ne semble pas avoir d'influence qualitative sur la prolifération de ces deux types de bactéries. Staphylococus epidermis sécrète l'élastase, responsable des lésions d'élastolyse périfolliculaire.

Les Pityrosporum, tel que ceux précédemment nommés, sont présents en des quantités du même ordre que les staphylocoques ou que les propionibactéries ; bien qu'aérobies, on peut les rencontrer dans les couches profondes du follicule ainsi que dans les comédons fermés.

Selon la directive du Conseil de la Communauté Economique Européenne N°76/768/CEE du 27 Juillet 1976 modifiée par la directive N°93/35/CEE du 14 Juin 1993, on entend par « produit cosmétique » toute substance ou préparation destinée à être mise en contact avec les diverses parties superficielles du corps humain (épiderme, système pileux et capillaire ongles, lèvres et organes génitaux) ou avec les dents et les muqueuses buccales en vue, exclusivement ou principalement, de les nettoyer, de les parfumer, d'en modifier l'aspect et/ou de corriger les odeurs corporelles et/ou de les protéger ou de les maintenir en bon état.

Du fait de la plus en plus grande prise en compte des problèmes de pollution liés à la vie moderne, notamment dans les lieux fortement urbanisés, l'aspect protection de la peau est devenu prépondérant dans la recherche de nouveaux produits cosmétiques. En réponse aux agressions ou aux sensations d'agression de la peau, on a développé le concept de produit cosmétique apaisant.

De manière générale, on appelle produit apaisant ou formulation cosmétique apaisante, tout produit en formulation qui procure une sensation de bien-être de la peau, que ce soit notamment une sensation de douceur, d'élasticité, et/ou de réconfort ressentie par le sujet grâce à l'application dudit produit sur sa peau.

Ils ont la particularité d'agir selon plusieurs mécanismes dermopharmacologiques, ce qui les rend très efficaces comme produits cosmétiques apaisants sur tous les types de peaux. C'est ainsi qu'ils possèdent à la fois une activité hydratante, une activité germicide et anti-microbienne, une activité anti-inflammatoire que ce soit en inhibant les radicaux libres formés notamment par le rayonnement ultraviolet ou en inhibant des enzymes, tels que les lipases, la lipooxygénase, la 5-alpha-réductase qui est notamment responsable de la production de sébum, l'élastase et la hyaluronidase qui sont notamment responsables de la dégradation de la matrice tissulaire et une activité antagoniste de la substance P.

Les composés à structure lipoaminoacide, comme par exemple ceux décrits dans les demandes internationales de brevet publiées sous les numéros WO92/20647, WO92/21318, WO94/26694 et WO94/27561, sont, en raison de leur structure amphiphile, des vecteurs biologiques particulièrement intéressants en tant que régulateurs de la physiologie cutanée et s'avèrent appropriés à de multiples applications, notamment en cosmétique.

Cependant, pour protéger les compositions cosmétiques ou dermopharmaceutiques des contaminations microbiennes, il est usuel d'y inclure des conservateurs chimiques comme le phénoxyéthanol et ses dérivés, le paraben et ses dérivés ou des composés qui, par décomposition lente, produisent du formol.

De tels composés présentent l'inconvénient d'entraîner chez certains utilisateurs des réactions d'intolérance.

Afin de réduire l'occurrence de tels effets secondaires, on a recherché à remplacer ces composés par d'autres moins irritants, sans pour autant affaiblir la protection des compositions cosmétiques contre les contaminations microbiennes.

La demande de brevet européen publiée sous le numéro EP 0 747 047 divulgue des associations de lipoaminoacides avec des monoalkyléthers de glycérol qui ne provoquent pas d'intolérance cutanée tout en présentant une activité anti-microbienne au moins aussi efficace que celle des compositions de l'art antérieur.

La demande de brevet allemand DE 22 03 228 A décrit une composition fongicide utilisée dans l'agriculture et l'horticulture, contenant comme composé actif de la N-undécylènoyl-L-leucine.

La demande de brevet japonais JP 49 093521 divulgue différents acides aminés N-acylés contenant le radical undécylènoyl, qui sont testés dans le traitement de maladies de peau. Parmi les aminoacides utilisés figurent la N-undécylènoylalanine, la N-undécylènoylcystine, la N-undécylènoylméthionine, la N-undécylènoylproline, l'acide N-undécylènoylglutamique, la N-undécylènoytserine et la N-undécylènoylvaline.

Dans le cadre de ses recherches pour améliorer la conservation des compositions cosmétiques, la demanderesse a développé de nouvelles compositions dont la protection anti-microbienne est potentialisée, par la présence de composés agissant en synergie, et dont le caractère apaisant n'est pas affecté.

L'invention a pour objet une composition comprenant un composé de formule (I) 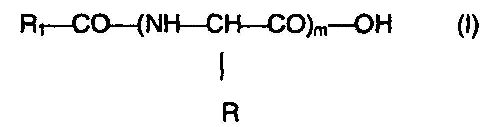 ou ses sels topiquement acceptables,
dans laquelle R₁-CO représente un radical octanoyle, R représente une chaîne caractérisante d'un acide aminé et m est compris entre 1 et 5, et au moins un composé de formule (II) 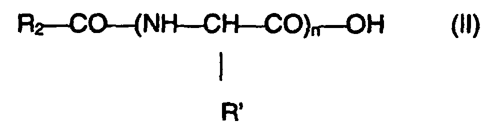 ou ses sels topiquement acceptables
dans laquelle R₂―CO représente un radical undécènoyle, R' représente une chaîne caractérisante d'un acide aminé et n est compris entre 1 et 5.

Par sel topiquement acceptable, on entend tout sel de l'acide de formule (I) ou de l'acide de formule (II) biologiquement acceptable pour la peau et/ou les muqueuses, c'est à dire tout sel pouvant notamment régler le pH de la composition à une valeur comprise entre 3 et 8 et de préférence environ égale à 5, c'est à dire à un pH voisin de celui de la peau.

Il peut s'agir notamment de sels alcalins tels que les sels de sodium, de potassium ou de lithium, de sels alcalino-terreux tels que les sels de calcium, de magnésium ou de strontium; il peut aussi s'agir de sels métalliques tels que les sels divalents de zinc ou de manganèse ou encore les sels trivalents de fer, de lantane, de cérium ou d'aluminium.

Les composés de formule (I) et de formule (II) présents dans la composition objet de la présente invention, peuvent être sous forme d'acides libres ou sous formes partiellement ou totalement salifiées.

L'expression « chaîne caractérisante » utilisée dans le cadre de la présente demande désigne la chaîne principale non fonctionnelle de l'acide aminé considéré.

Pour un acide aminé représenté par la formule générale

H₂N-CHR-COOH,

la chaîne caractérisante sera la chaîne représentée par R.

R et R' représentent indépendamment l'un de l'autre notamment la chaîne caractérisante d'un des acides aminés choisis parmi la glycine, l'alanine, la sérine, l'acide aspartique, l'acide glutamique, la valine, la thréonine, l'arginine, la lysine, la praline, la leucine, la phénylalanine, l'isoleucine, l'histidine, la tyrosine, le tryptophane, l'asparagine, la cystéine, la cystine, la méthionine, l'hydroxyproline, l'hydroxylysine et l'omithine.

L'invention a plus particulièrement pour objet la composition telle que décrite précédemment pour laquelle, dans les formules (I),et (II) ; R et R' représentent indépendamment l'un de l'autre la chaîne caractérisante de la glycine, de l'alanine, de l'acide glutamique ou de l'acide aspartique.

Les composés de formule (I) et de formule (II) sont généralement obtenus par acylation de composés de formule (I_{A}) 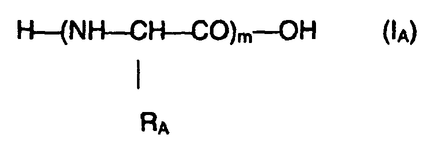 ou de leur sels, dans laquelle R_{A} représente un des radicaux R ou R' tels que définis précédemment, eux-mêmes obtenus par hydrolyse totale ou partielle de protéines de toutes origines. Ces protéines peuvent être d'origine animale, telles que, par exemple, le collagène, l'élastine, la protéine de chair de poissons, la gélatine de poissons, la kératine ou la caséine, d'origine végétale, telles que, par exemple, celles issues du soja, du tournesol, de l'avoine, du blé, du maïs, de l'orge, de la pomme de terre, du lupin, de la féverolle, de l'amande douce, de la soie, ou encore obtenues à partir de chorelles (algues unicellulaires), d'algues roses ou de levures.

Cette hydrolyse peut être réalisée par exemple par chauffage à des températures comprises entre 60 et 130°C d'une protéine placée dans un milieu acide ou alcalin.

Cette hydrolyse peut également être réalisée par voie enzymatique avec une protéase, couplée éventuellement à une post-hydrolyse alcaline ou acide.

Quand m et/ou n est supérieur à 1, R et/ou R' représentent plusieurs des chaînes caractérisantes des acides aminés, selon la protéine hydrolysée et le degré d'hydrolyse.

Dans une variante préférée de la présente invention, lorsque la composition ne comprend qu'un seul composé de formule (II) et un seul composé de formule (I), m et n sont égaux à 1 et lorsque la composition comprend un mélange de composés de formule (II) et éventuellement un mélange de composés de formule (I), les degrés moyens de condensation des acides aminés N-acylés dans ces mélanges sont inférieurs à 2.

La réaction d'acylation permettant d'obtenir les composés de formules (I) et (II) précitées peut être réalisée par voie chimique en milieu alcalin (pH de 8 à 10) selon la réaction de Schotten Bauman ou par voie enzymatique et l'homme de métier pourra se reporter notamment à la référence Surfactant Science Series, volume 7, Anionic Surfactants, partie II, chapitre 16, pages 581 à 617 (Marcel Dekker - 1976).

D'une façon générale, le mode de réalisation actuellement préféré pour la préparation des composés lipoaminoacides de formules (I) et (II) comprend les étapes suivantes :
**a)** Acylation en milieu alcalin (pH 8 à 10) d'un excès de mélange d'acides aminés (mélange extemporané ou obtenu par hydrolyse complète d'une protéine) par un acide gras (ou un mélange d'acides gras), sous forme de chlorure d'acide ou d'anhydride.
   Le rapport acides aminés/chlorure acide est de préférence de 1,05 à 1,30 équivalents.
   La température d'acylation optimale se situe vers 80° C mais varie d'un acide aminé à l'autre entre 60 et 110° C.
   La durée d'acylation dépend de l'équipement utilisé (taille, agitation); elle est de 2 heures environ pour une masse acylée de 500 kg et de 5 heures environ pour une masse acylée de 5 000 kg.
**b)** Cassage de l'acylat alcalin par acidification pour décanter les impuretés solubles dans l'eau et relarguer l'acylat organique acide (pH optimal de 0,5 à 3 selon les acides aminés).
**c)** Purification par lavage à l'eau ou avec addition d'électrolytes ou de co-solvant pour favoriser la décantation.

Outre les principes actifs, de formule (I) et de formule (II), la composition selon l'invention comprend des véhicules minéraux ou organiques couramment utilisés dans la fabrication de compositions destinées à être formulées en préparations à usage cosmétique et/ou pharmaceutique : on peut citer par exemple l'eau ou les mélanges eau/alcool tels que les solutions aqueuses d'éthanol, de propanol ou d'isopropanol.

Dans un aspect préféré de la présente invention la composition telle que décrite précédemment comprend de 15 % à 60% et, plus particulièrement, de 20 % à 40 % en poids d'un mélange d'au moins un composé de formule (I) et d'au moins un composé de formule (II) ou leurs sels topiquement acceptables, tel que le rapport pondéral composé de formule (I)/composé e formule (II) est inférieur ou égal à 10 et supérieur ou égal à 0,1, et est de préférence inférieur ou égal à 5 et supérieur ou égal à 0,2.

Dans une variante particulière de la présente invention, le rapport pondéral composé de formule (I) / composé de formule (II) est supérieur ou égal à 0,4 et inférieur ou égal à 2,5 et est de préférence égal à 1.

La composition objet de la présente invention peut aussi comprendre, outre le mélange principe actif de formule (II) et principe actif de formule (I), tels que définis précédemment, des composés à activité germicide, et/ou d'autres composés à activité apaisante et/ou inflammatoire.

Dans une autre variante de la présente invention, la composition comprend outre le mélange de composés de formule (I) et de formule (II), tels que définis précédemment, un ou plusieurs composés choisis parmi le gluconate de zinc, les constituants d'un extrait ou d'une teinture de matières végétales riches en tannins, ou l'aspartate mixte de magnésium et de potassium,
ou les composés de formule (III)

R₃―CHOH―CH₂OH (III)

ou leurs sels topiquement acceptables,
dans laquelle R₃ représente un radical aliphatique linéaire ou ramifié, saturé ou insaturé, comportant de 6 à 16 atomes de carbone tel que l'octanediol-1,2.

Dans la définition précédente, les mots « extrait » et « teinture » sont utilisés dans leurs sens respectifs tels qu'ils sont établis dans l'édition de 1997 de la Pharmacopée Européenne ; les extraits (extracts) sont des préparations concentrées, liquides, solides ou de consistance intermédiaire, généralement obtenues à partir de matières premières végétales ou animales séchées. Les teintures (tincturae) sont des préparations généralement obtenues à partir de matières premières végétales ou animales séchées.

Par « extraits » ou « teintures » de matières premières végétales riches en tannins, on désigne notamment les extraits ou teintures de rathania, de thé, de cannelle, de saules ou de hamamelis. Ces extraits ou teintures sont disponibles dans le commerce. Certains sont inscrits aux pharmacopées française et/ou européenne.

Dans une variante préférée de la présente invention, lorsque la composition telle que définie précédemment comprend un extrait ou une teinture de matières végétales riches en tannins, il s'agit d'un extrait de cannelle.

Lorsque de l'aspartate mixte de magnésium et potassium est présent dans la composition objet de la présente invention, cette composition en contient 0,5 % à 5 % en poids.

Lorsque le gluconate de zinc est présent dans la composition objet de la présente invention, cette composition en contient de 0,1 % à 10 % en poids et, plus particulièrement, de 0,5 % à 5 % en poids.

Lorsqu'un ou plusieurs composés de formule (III) est présent dans la composition objet de la présente invention, cette composition en contient de 15 à 60 % en poids.

Lorsqu'un ou plusieurs extraits ou teintures de matières végétales riches en tannins sont présents dans la composition objet de la présente invention, cette composition en contient de 0,1 % à 10 % en poids et, plus particulièrement de 0,5 % à 5 % en poids.

Selon la Pharmacopée Européenne, les extraits peuvent être sous forme d'extraits fluides, d'extraits mous ou fermes ou d'extraits secs.

Dans la définition précédente de la composition selon l'invention, les pourcentages pondéraux en constituants de l'extrait ou de la teinture correspondent aux pourcentages pondéraux en résidu sec, ledit résidu sec étant obtenu par évaporation du solvant et dessiccation dudit extrait ou de ladite teinture à des conditions opératoires auxquelles l'altération des constituants est minime.

La composition selon l'invention est utilisée en cosmétique. Comme le montrent les exemples suivants, la composition selon l'invention se caractérise de façon inattendue par une activité anti-microbienne accrue à pH5. Ces exemples démontrent notamment que l'undécylénoyl glycine présente un large spectre d'activités bactériostatiques et de fongistatiques. Ils montrent aussi que l'association de l'octanoyl glycine avec l'undécylénoyl glycine permet de formuler des produits sans conservateur ou au moins de réduire nettement les concentrations en conservateurs chimiques. Ceci permet de l'utiliser notamment, pour apaiser et/ou protéger les peaux sensibles, hydrater les peaux sèches, ralentir le vieillissement de la peau et /ou traiter les peaux à tendance acnéique et/ou les peaux séborrhéiques ; la composition selon l'invention peut donc être utilisée comme traitement complémentaire au traitement médical de l'acné.

La composition cosmétique telle que définie précédemment comprenant de l'undécylénoyl glycine et de l'octanoyl glycine est particulièrement appropriée à la restauration du manteau acide de la peau.

En effet, il est reconnu que les peaux saines présentent un pH acide, dû essentiellement à la présence de lipoprotéines naturelles d'origine métabolique. Or, les diverses agressions provoquent très souvent une décarboxylation de ces lipoprotéines conduisant à une basification du pH cutané par formation d'amines toxiques pour la peau. Les peaux « malades » ont alors un pH plus basique que les peaux « normales ». Cette modification du pH cutané est souvent suivie d'une perturbation de la flore saprophyte de la peau, qui peut s'appauvrir au bénéfice de la prolifération de germes pathogènes par exemple.

Les exemples exposés ci-après montrent que l'undécylénoyl glycine participe à la restauration du manteau acide de la peau de façon beaucoup plus efficace que l'acide lactique généralement utilisé.

La composition selon l'invention est aussi utilisée pour la désinfection de la peau et des muqueuses. Dans ce cas il peut s'agir d'un simple acte d'hygiène corporelle ou d'un traitement complémentaire au traitement médical d'une infection.

La composition selon l'invention est aussi utilisée dans le traitement du cuir chevelu notamment comme actif antipelliculaire.

La composition cosmétique telle que définie précédemment comprenant de l'undécylénoyl glycine et de l'octanoyl glycine est particulièrement appropriée au traitement anti-pelliculaire, ainsi que le montrent les exemples exposés ci-après.

Selon l'utilisation, la composition telle que décrite précédemment est mise en oeuvre à des concentrations différentes et dans une formulation appropriée à cette utilisation; de telles compositions cosmétiques se présentent habituellement sous forme de solutions aqueuses, de solutions alcooliques diluées, ou d'émulsions simples ou multiples, telles que les émulsions eau dans huile (E/H), huile dans eau (H/E) ou eau dans huile dans eau (E/H/E). Comme formulation cosmétique on peut citer, les crèmes, les laits, les lotions, les toniques, les lingettes, les gels douches, les savons, les savons liquides, les syndets, les produits d'hygiène intime, ou les shampooings, les produits déodorants, les produits de maquillage, ou les produits démaquillants.

De telles formulations sont connues de l'homme du métier; leur préparation sont décrites, par exemple, dans les demandes de brevet publiées sous les numéros, WO92/06778, WO93/28204, WO95/13863, WO95/35089, WO96/22109, WO 98/09611, WO 98/22207, WO 96/37285 ou WO98/47610.

L'invention a donc aussi pour objet une formulation cosmétique susceptible d'être obtenue par dilution du 1/10 jusqu'au 1/20000 de la composition telle que décrite précédemment, dans un ou plusieurs excipients cosmétiquement acceptables, et notamment une formulation cosmétique sous forme d'une émulsion huile dans eau ayant l'aspect d'un lait ayant une viscosité inférieure à 1Pa.s., comprenant comme émulsionnant, une composition auto-émulsionnable à base d'alcools gras.

Comme composition auto-émulsionnable préférée, on peut par exemple citer le MONTANOV™ 68 ou le MONTANOV™ 202 commercialisé par la société SEPPIC.

Le terme dilution employé dans ce qui précède, englobe dans son acception la plus large, toutes les étapes permettant de passer de la composition telle que définie précédemment à la formulation cosmétique destinée à être commercialisée.

Dans un autre mode préféré de la présente invention, la formulation cosmétique est une lotion pour traiter les peaux à tendance acnéique.

Dans un autre mode préféré de la présente invention, la formulation cosmétique est une formule moussante ou un shampooing antipelliculaire.

L'invention a particulièrement pour objet une formulation cosmétique comprenant de 0,001% à 6 % en poids d'au moins un composé de formule (1) et de 0,001 % à 6 % en poids d'au moins un composé de formule (II), désiré jusqu'à 1 % en poids d'au moins un extrait ou une teinture de matières premières végétales riches en tannins choisi parmi les extraits de cannelle, de rathania, de thé, de saules, ou de hamamelis, si désiré jusqu'à 1% de gluconate de zinc.

L'invention a tout particulièrement pour objet une formulation cosmétique comprenant comme principe actif de 0,5 % à 5 % en poids d'un composé de formule (I₁)

R₁―CO―NH―CH₂―COOH (I₁)

ou un de sels topiquement acceptables et de 0,5 % à 5 % en poids d'un composé de formule (II₁)

R₂―CO―NH―CH₂COOH (II₁)

ou de ses sels topiquement acceptables
formules (I) et (II) dans lesquelles R₁―CO et R₂―CO sont tels que définis précédemment, si désiré de 0,5 % à 2 % en poids d'octanediol, si désiré, jusqu'à 0,2 % en poids de constituants d'un extrait de matière première végétale choisis parmi les extraits de cannelle, de rathania, de thé, de saules, ou de hamamelis, si désiré jusqu'à 0,2 % en poids de gluconate de zinc, et si désiré jusqu'à 0,2 % en poids d'aspartate mixte de potassium et de sodium.

### EXEMPLES

### A) PREPARATION DES COMPOSITIONS SELON L'INVENTION

**a)** On mélange sous agitation les composés suivants :
- Lipacide™ UG, commercialisé par la Société SEPPIC dont le principe actif est l'undécylénoyl glycine,
- Lipacide™ C8G, commercialisé par la Société SEPPIC, dont le principe actif est l'octanoyl glycine.
- Octanediol SJ®,
- Glycérine,
- Tris (Tris hydroxyméthyl aminométhane ou trométhamine) en quantité suffisante pour obtenir un pH d'environ 5,0 à 6,0
- eau

On obtient une solution liquide, stable, inodore et hydrosoluble dont le pH est d'environ 5,6 à 6.0.
Après filtration sur un filtre à membrane (environ 3η), on obtient une composition A contenant environ :
- 12,5 % en poids de Lipacide™ UG
- 12,5 % en poids de Lipacide™ C 8G
- 10 % en poids de glycérine.

**b)** En mettant en oeuvre le procédé décrit en a), mais en ajoutant avant filtration sur membrane, une solution aqueuse d'un extrait sec de cannelle commercialisé par la Société ALBAN MULLER INTERNATIONAL, on obtient respectivement, la composition A₁, contenant environ :

| | |
|---|---|
| Composition A₁ | 12,5 % en poids de Lipacide™ C8G |
| | 12,5 % en poids de Lipacide™ UG |
| | 10 % en poids de glycérine |
| | 3 % en poids de constituants de l'extrait sec de cannelle |

### B) MISE EN EVIDENCE DE L'ACTIVITE ANTI-MICROBIENNE DES COMPOSITIONS SELON L'INVENTION

On a déterminé les CMI en milieu liquide, avec un temps d'incubation de 72 heures à 30°C, à pH 7 (bouillon Trypticase soja tamponné), du Lipacide™ UG seul, du conservateur testé seul, ainsi que du mélange des deux produits La comparaison des résultats consignés dans les tableaux suivants fait apparaître nettement une synergie anti-microbienne qui autorise ainsi une réduction de la concentration nécessaires en conservateurs chimique dans les compositions.

La composition A est diluée dans l'eau pour obtenir la composition A₂ contenant 0,05 % en poids d'undécylènoyl glycine et 0,05 % en poids d'octanoyl glycine et la composition A₃ contenant 0,125 % en poids d'undécylènoyl glycine et 0,125 % en poids d'octanoyl glycine.

On obtient les résultats suivants (CMI à pH5).

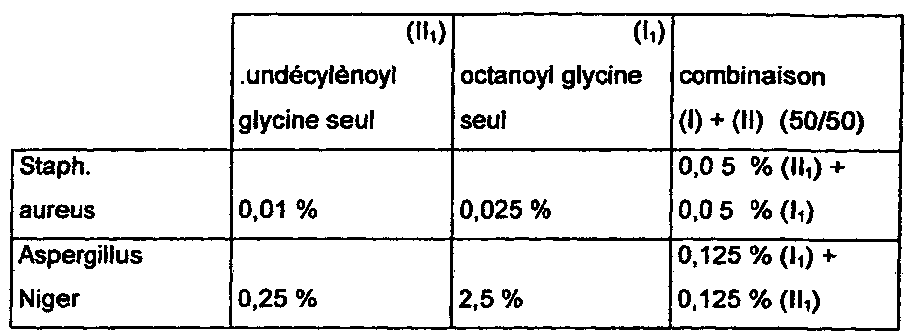

Ces résultats font apparaître une synergie de l'activité anti-microbienne inhérente aux combinaisons des produits (I) + (II), que ce soit sur des bactéries gram +, ou sur des champignons.

## Revendications

1. Composition **caractérisée en ce qu'**elle comprend un composé de formule (I) : 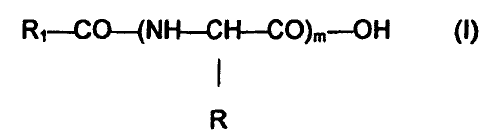 ou ses sels topiquement acceptables,
dans laquelle R₁-CO représente un radical octanoyle, R₁ représente une chaîne caractérisante d'un acide aminé et m est compris entre 1 et 5, et au moins un composé de formule (II) 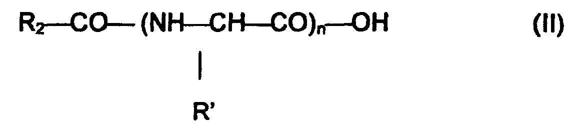 ou ses sels topiquement acceptables,
dans laquelle R₂-CO représente un radical undécylènoyle, R' représente une chaîne caractérisante d'un acide aminé et n est compris entre 1 et 5.

2. Composition telle que définie à la revendication 1, pour laquelle, dans les formules (I) et (II), R et R' représentent indépendamment l'un de l'autre la chaîne caractérisante de la glycine, de l'alanine, de l'acide glutamique ou de l'acide aspartique.

3. Composition telle que définie à l'une quelconque des revendications 1 ou 2, **caractérisée en ce que** lorsque la composition ne comprend qu'un seul composé de formule (II) et un seul composé de formule (1), m et n sont égaux à 1 et lorsque la composition comprend un mélange de composés de formule (I), les degrés moyens de condensation des acides aminés N-acylés dans ces mélanges sont inférieurs à 2.

4. Composition telle que définie à l'une quelconque des revendications 1 à 3, comprenant de 15 % à 60 % et notamment de 20 à 40 % en poids d'un mélange d'au moins un composé de formule (I) ou un de ses sels topiquement acceptables, et de 15 % à 60 % et notamment de 20 % à 40 % en poids d'au moins un composé de formule (II) ou un de ses sels topiquement acceptables, tel que rapport pondéral composé de formule (I)/composé de formule (II) est inférieur ou égal à 10 et supérieur ou égal à 0,1, et est de préférence inférieur ou égal à 5 et supérieur ou égal à 0,2.

5. Composition telle que définie à la revendication 4, pour laquelle le rapport pondéral composé de formule (I) / composé de formule (II) est supérieur ou égal à 0,4 et inférieur ou égal à 2,5 et est de préférence égal à 1.

6. Composition telle que définie à l'une des revendications 1 à 3, comprenant éventuellement en poids d'octanoyl glycine et de 20 % à 40 % en poids d'undécylènoyl glycine.

7. Composition telle que définie à l'une quelconque des revendications 1 à 6, **caractérisée en ce qu'**elle comprend en outre de 0,1 % à 10 % en poids et de préférence de 0,5 à 5 % de gluconate de zinc.

8. Composition telle que définie à l'une quelconque des revendications 1 à 7, **caractérisée en ce qu'**elle comprend en outre de 0,1 % à 10 % en poids et notamment 0,5 % à 5 % en poids d'au moins un extrait et/ou d'au moins une teinture de matières premières végétales riches en tannins.

9. Composition telle que définie à la revendication 8 dans laquelle l'extrait de matières végétales riches en tannins est un extrait de cannelle.

10. Composition telle que définie à l'une quelconque des revendications 1 à 9 **caractérisée en ce qu'**elle comprend en outre de 0,5 % à 5 % en poids d'aspartate mixte de magnésium et de potassium.

11. Composition telle que définie à l'une des revendications 1 à 12 **caractérisée en ce qu'**elle comprend en outre de 15 % à 60 % en poids et de préférence de 20 à 40 % d'un composé de formule (III)
R₃―CHOH―CH₂OH (III)
ou leurs sels topiquement acceptables,
dans laquelle R₃ représente un radical aliphatique linéaire ou ramifié, saturé ou insaturé, comportant de 6 à 16 atomes de carbone tel que l'octanediol-1,2.

12. Utilisation de la composition telle que définie à l'une des revendications 1 à 11 en cosmétique.

13. Utilisation selon la revendication 12 pour apaiser /ou protéger les peaux sensibles, pour hydrater les peaux sèches, et/ou pour ralentir le vieillissement de la peau, et/ou traiter le cuir chevelu, et/ou pour restaurer le manteau acide de la peau.

14. Utilisation selon la revendication 13 pour désinfecter la peau et les muqueuses.

15. Utilisation selon la revendication 14 pour traiter les peaux à tendance acnéique et/ou les peaux sébborhéiques.

16. Formulation cosmétique susceptible d'être obtenue par dilution du 1/10 jusqu'au 1/20000 de la composition telle que définie par l'une quelconque des revendications 1 à 11, dans un ou plusieurs excipients cosmétiquement acceptables.

17. Formulation telle que définie à la revendication 16 sous forme d'une émulsion huile dans eau ayant l'aspect d'un lait ayant une viscosité inférieure à 1 Pa.s. comprenant comme émulsionnant une composition auto-émulsionnable à base d'alcools gras.

18. Formulation telle que définie à la revendication 16, sous forme d'une lotion pour traiter les peaux à tendance acnéique.

19. Formulation telle que définie à la revendication 16 sous forme d'une formule moussante ou un shampooing antipelliculaire.

20. Formulation cosmétique comprenant à titre de principe actif de 0,001 à 6 % en poids d'au moins un composé de formule (I) et de 0,001 % à 6% en poids d'au moins un composé de formule (II), désiré jusqu'à 1 % en poids d'au moins un extrait et/ou d'au moins une teinture de matières premières végétales riches en tannins choisis parmi les extraits de cannelle, de rathania, de thé, de saules ou de hamamélis si désiré jusqu'à 1 % de gluconate de zinc.

21. Formulation cosmétique comprenant comme principe actif de 0,5 % à 5 % en poids d'un composé de formule (I₁)
R₁―CO―NH―CH₂―COOH (I₁)
ou un de ses sels cosmétiquement acceptables et de 0,5 % à 5 % en poids d'un composé de formule (II₁)
R₂―CO―NH―CH₂COOH (II₁)
ou un de ses sels cosmétiquement acceptables,
formules (I₁) et (II₂) dans lesquelles les groupes R₁CO et R₂CO sont tels que définis à la revendication 1, de si désiré 0,5 % à 2 % en poids de constituants d'un extrait de matières premières végétales choisi parmi les extraits de cannelle, de rathania, de thé, de saule, ou de hamamelis, si désiré jusqu'à 0,2 % en poids de gluconate de zinc, et si désiré jusqu'à 0,2 % d'aspartate mixte de potassium et de magnésium.

## Patentansprüche

1. Zusammensetzung, **dadurch gekennzeichnet, daß** sie eine Verbindung der Formel (I) 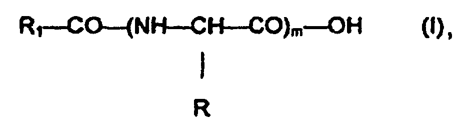 in der R₁-CO einen Octanoylrest bedeutet, R₁ eine für eine Aminosäure charakteristische Kette bedeutet und m 1 bis 5 bedeutet,
oder ihre topisch unbedenklichen Salze, sowie mindestens eine Verbindung der Formel (II) 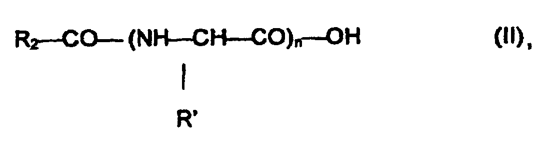 in der R₂-CO einen Undecylenoylrest bedeutet, R' eine für eine Aminosäure charakteristische Kette bedeutet und n 1 bis 5 bedeutet,
oder ihre topisch unbedenklichen Salze, enthält.

2. Zusammensetzung nach Anspruch 1, in der in den Formeln (I) und (II) R und R' unabhängig voneinander die für Glycin, Alanin, Glutaminsäure oder Asparaginsäure typische Kette bedeuten.

3. Zusammensetzung nach Anspruch 1 oder 2, **dadurch gekennzeichnet, daß**, wenn die Zusammensetzung nur eine einzige Verbindung der Formel (II) und eine einzige Verbindung der Formel (I) enthält, m und n 1 bedeuten, und wenn die Zusammensetzung eine Mischung aus Verbindungen der Formel (I) enthält, die mittleren Kondensationsgrade der N-Acyl-Aminosäuren in diesen Mischungen unter 2 betragen.

4. Zusammensetzung nach einem der Ansprüche 1 bis 3, die 15 Gew.-% bis 60 Gew.-%, insbesondere 20 bis 40 Gew.-%, einer Mischung aus mindestens einer Verbindung der Formel (I) oder einem ihrer topisch unbedenklichen Salze und 15 Gew.-% bis 60 Gew.-%, insbesondere 20 Gew.-% bis 40 Gew.-%, mindestens einer Verbindung der Formel (II) oder eines ihrer topisch unbedenklichen Salze enthält, so daß das Gewichtsverhältnis Verbindung der Formel (I)/Verbindung der Formel (II) höchstens 10 und mindestens 0,1, vorzugsweise höchstens 5 und mindestens 0,2 beträgt.

5. Zusammensetzung nach Anspruch 4, bei der das Gewichtsverhältnis Verbindung der Formel (I)/Verbindung der Formel (II) mindestens 0,4 und höchstens 2,5, vorzugsweise 1, beträgt.

6. Zusammensetzung nach einem der Ansprüche 1 bis 3, die gegebenenfalls ... Gewichts... Octanoylglycin und 20 Gew.-% bis 40 Gew.-% Undecylenoylglycin enthält.

7. Zusammensetzung nach einem der Ansprüche 1 bis 6, **dadurch gekennzeichnet, daß** sie weiterhin 0,1 Gew.-% bis 10 Gew.-%, vorzugsweise 0,5 bis 5 Gew.-%, Zinkgluconat enthält.

8. Zusammensetzung nach einem der Ansprüche 1 bis 7, **dadurch gekennzeichnet, daß** sie weiterhin 0,1 Gew.-% bis 10 Gew.-%, insbesondere 0,5 Gew.-% bis 5 Gew.-%, an mindestens einem Extrakt und/oder mindestens einer Tinktur aus gerbstoffreichen pflanzlichen Ausgangsmaterialien enthält.

9. Zusammensetzung nach Anspruch 8, bei der der Extrakt aus gerbstoffreichen Pflanzenmaterialien ein Zimtextrakt ist.

10. Zusammensetzung nach einem der Ansprüche 1 bis 9, **dadurch gekennzeichnet, daß** sie weiterhin 0,5 Gew.-% bis 5 Gew.-% Asparaginsäure-Magnesium- und -Kaliummischester enthält.

11. Zusammensetzung nach einem der Ansprüche 1 bis 12, **dadurch gekennzeichnet, daß** sie weiterhin 15 Gew.-% bis 60 Gew.-%, vorzugsweise 20 bis 40 Gew.-%, einer Verbindung der Formel (III)
**R**_{**3**}**―CHOH―CH**_{**2**}**OH (III),**
in der R₃ einen geradkettigen oder verzweigten, gesättigten oder ungesättigten aliphatischen Rest mit 6 bis 16 Kohlenstoffatomen, wie Octan-1,2-diol, bedeutet,
oder ihre topisch unbedenklichen Salze enthält.

12. Verwendung der Zusammensetzung nach einem der Ansprüche 1 bis 11 in der Kosmetik.

13. Verwendung nach Anspruch 12 zur Beruhigung und/oder zum Schutz von empfindlicher Haut, zur Hydratisierung von empfindlicher Haut und/oder zur Verlangsamung der Hautalterung und/oder zur Behandlung der Kopfhaut und/oder zur Wiederherstellung des Säureschutzmantels der Haut.

14. Verwendung nach Anspruch 13 zur Desinfektion der Haut und der Schleimhäute.

15. Verwendung nach Anspruch 14 zur Behandlung von Haut, die zu Akne neigt, und/oder von fetter Haut.

16. Kosmetikformulierung, die dadurch erhalten werden kann, daß man die Zusammensetzung nach einem der Ansprüche 1 bis 11 im Verhältnis 1/10 bis 1/20 000 mit einem oder mehreren kosmetisch unbedenklichen Trägerstoffen verdünnt.

17. Formulierung nach Anspruch 16 in Form einer milchartigen Öl-in-Wasser-Emulsion mit einer Viskosität unter 1 Pa.s, die als Emulgator eine selbstemulgierbare Zusammensetzung auf Fettalkoholbasis enthält.

18. Formulierung nach Anspruch 16 in Form einer Lotion zur Behandlung von Haut, die zu Akne neigt.

19. Formulierung nach Anspruch 16 in Form eines schäumenden Produkts oder eines Shampoos gegen Schuppen.

20. Kosmetikformulierung, die als Wirkstoff 0,001 bis 6 Gew.-% an mindestens einer Verbindung der Formel (I) und 0,001 Gew.-% bis 6 Gew.-% an mindestens einer Verbindung der Formel (II) sowie gewünschtenfalls bis 1 Gew.-% an mindestens einem Extrakt und/oder mindestens einer Tinktur aus gerbstoffreichen pflanzlichen Ausgangsmaterialien aus der Gruppe der Zimt-, Ratanhia-, Tee-, Weidenoder Zaubernußextrakte sowie gegebenenfalls bis zu 1% Zinkgluconat enthält.

21. Kosmetikformulierung, die als Wirkstoff 0,5 Gew.-% bis 5 Gew.-% einer Verbindung der Formel (I₁)
**R**_{**1**}**―CO―NH―CH**_{**2**}**―COOH (I**_{**1**}**)**
oder eines ihrer kosmetisch unbedenklichen Salze sowie 0,5 Gew.-% bis 5 Gew.-% einer Verbindung der Formel (II₁)
**R**_{**2**}**―CO―NH―CH**_{**2**}**COOH (II**_{**1**}**)**
oder eines ihrer kosmetisch unbedenklichen Salze, wobei in den Formeln (I₁) und (II₁) die Gruppen R₁CO und R₂CO wie in Anspruch 1 definiert sind, sowie gewünschtenfalls 0,5 Gew.-% bis 2 Gew.-% Bestandteile eines Extrakts aus pflanzlichen Ausgangsmaterialien aus der Gruppe der Zimt-, Ratanhia-, Tee-, Weiden- oder Zaubernußextrakte sowie gewünschtenfalls bis zu 0,2 Gew.-% Zinkgluconat und gewünschtenfalls bis zu 0,2% Asparaginsäure-Kalium- und -Magnesium-Mischester enthält.

## Claims

1. Composition, **characterized in that** it comprises a compound of the formula (I): 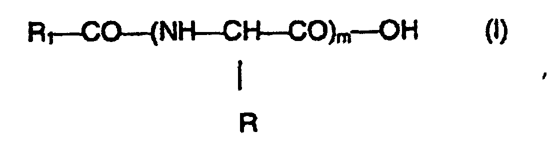 in which R₁-CO represents an octanoyl radical, R₁ represents a chain which is characteristic of an amino acid and m is between 1 and 5,
or its topically acceptable salts,
and at least one compound of the formula (II) 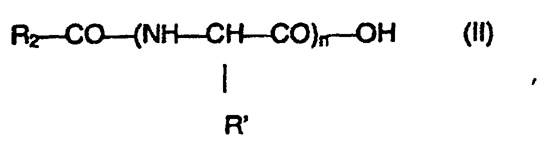 in which R₂-CO represents an undecylenoyl radical, R' represents a chain which is characteristic of an amino acid and n is between 1 and 5,
or its topically acceptable salts.

2. Composition as defined in Claim 1, where in the formulae (I) and (II), R and R' independently of one another represent the chain which is characteristic of glycine, alanine, glutamic acid or aspartic acid.

3. Composition as defined in Claim 1 or 2, **characterized in that**, when the composition comprises only a single compound of the formula (II) and a single compound of the formula (I), m and n equal 1, and when the composition comprises a mixture of compounds of the formula (I), the mean degrees of condensation of the N-acyl-amino acids in these mixtures are less than 2.

4. Composition as defined in any of Claims 1 to 3, comprising 15% to 60%, in particular 20 to 40% by weight, of a mixture of at least one compound of the formula (I) or one of its topically acceptable salts, and 15% to 60%, in particular 20% to 40% by weight, of at least one compound of the formula (II) or one of its topically acceptable salts, so that the weight ratio compound of the formula (I)/compound of the formula (II) is not more than 10 and not less than 0.1, preferably not more than 5 and not less than 0.2.

5. Composition as defined in Claim 4, in which the weight ratio compound of the formula (I)/compound of the formula (II) is 0.4 or above and 2.5 or less, preferably 1.

6. Composition as defined in any of Claims 1 to 3, which optionally comprises by weight of octanoyl glycine and 20% to 40% by weight of undecylenoyl glycine.

7. Composition as defined in any of Claims 1 to 6, **characterized in that** it furthermore comprises 0.1% to 10% by weight, preferably 0.5 to 5%, of zinc gluconate.

8. Composition as defined in any of Claims 1 to 7, **characterized in that** it furthermore comprises 0.1% to 10% by weight, in particular 0.5% to 5% by weight, of at least one extract and/or at least one tincture of plant-based starting materials which are high in tannins.

9. Composition as defined in Claim 8, in which the extract of plant-based materials which are high in tannins is a cinnamon extract.

10. Composition as defined in any of Claims 1 to 9, **characterized in that** it furthermore comprises 0.5% to 5% by weight of magnesium potassium mixed aspartate.

11. Composition as defined in any of Claims 1 to 12, **characterized in that** it furthermore comprises 15% to 60% by weight, preferably 20 to 40% by weight, of a compound of the formula (III)
R₃-CHOH-CH₂OH (III),
in which R₃ represents a linear or branched, saturated or unsaturated aliphatic radical having 6 to 16 carbon atoms, such as octane-1,2-diol, or its topically acceptable salts.

12. Use of the composition as defined in any of Claims 1 to 11 in cosmetology.

13. Use according to Claim 12, for soothing and/or protecting sensitive skin, for hydrating dry skin, and/or for slowing down the ageing process of the skin, and/or for treating the scalp, and/or for restoring the protective acid pH of the skin.

14. Use according to Claim 13, for disinfecting the skin and the mucous membranes.

15. Use according to Claim 14, for treating acne-prone skin and/or greasy skin.

16. Cosmetic formulation which can be obtained by diluting the composition as defined in any of Claims 1 to 11 in one or more cosmetically acceptable excipients at a ratio of 1/10 to 1/20 000.

17. Formulation as defined in Claim 16, in the form of a milky oil-in-water emulsion with a viscosity of less than 1 Pa.s which contains, as emulsifier, a self-emulsifiable composition based on fatty alcohols.

18. Formulation as defined in Claim 16, in the form of a lotion for treating acne-prone skin.

19. Formulation as defined in Claim 16, in the form of a foaming formula or a shampoo with anti-dandruff action.

20. Cosmetic formulation which comprises, as active principle, 0.001 to 6% by weight of at least one compound of the formula (I) and 0.001% to 6% by weight of at least one compound of the formula (II), if desired up to 1% by weight of at least one extract and/or one tincture of plant-based starting materials which are high in tannins from the group of the cinnamon, rhatany, tea, willow or witchhazel extracts and, if desired, up to 1% of zinc gluconate.

21. Cosmetic formulation which comprises, as active principle, 0.5% to 5% by weight of a compound of the formula (I₁)
R₁-CO-NH-CH₂-COOH (I₁),
or of one of its cosmetically acceptable salts and 0.5% to 5% by weight of a compound of the formula (II₁)
R₂-CO-NH-CH₂COOH (II₁),
or one of its cosmetically acceptable salts,
where, in formulae (I₁) and (II₁), the groups R₁CO and R₂CO are as defined in Claim 1, if desired 0.5% to 2% by weight of constituents of an extract of plant-based starting materials selected from the group of the cinnamon, rhatany, tea, willow or witchhazel extracts, if desired, up to 0.2% of zinc gluconate and if desired up to 0.2% of potassium magnesium mixed aspartate.
